# EUROPEAN PATENT SPECIFICATION

(11) **EP 3 500 263 B1**
(45) Date of publication and mention of the grant of the patent: **21.09.2022**
(21) Application number: 18813239.3
(22) Date of filing: 04.06.2018
(51) Int. Cl.: C07K 14/47, C07K 14/82, C12N 9/12, A61K 31/505, A61K 31/551, A61P 35/00

(54) **PROCESS FOR EXPLOITING SYNTHETIC LETHALITY BASED ON OVEREXPRESSION OF MYC ONCOGENE**
VERFAHREN ZUR AUSNUTZUNG VON SYNTHETISCHER LETALITÄT AUF DER BASIS VON ÜBEREXPRESSION DES MYC-ONKOGENS
PROCÉDÉ D'EXPLOITATION DE LÉTALITÉ SYNTHÉTIQUE BASÉE SUR LA SUREXPRESSION D'ONCOGÈNE MYC

(30) Priority: 04.06.2017 US 201762514861 P
(43) Date of publication of application: 26.06.2019
(73) Proprietor: Chengdu Anticancer Bioscience, Ltd., Chengdu (CN)
(72) Inventor: ZHANG, Jing, Leuchars Fife KY16 OHP (GB); ZHANG, Shenqiu, St. Andrews Fife KY16 8XN (GB); YANG, Dun, South San Francisco, California 94080 (US)
(74) Representative: Lee, Nicholas John
(86) International application number: PCT/US2018/035907
(87) International publication number: WO 2018/226603

(56) References cited:
- WO-A1-2017/079442
- US-A1- 2002 111 289
- US-A1- 2015 031 683
- R. RATTAN ET AL: "Rho/ROCK pathway as a target of tumor therapy", JOURNAL OF NEUROSCIENCE RESEARCH., vol. 83, no. 2, 1 February 2006 (2006-02-01), pages 243-255, XP055557616, US ISSN: 0360-4012, DOI: 10.1002/jnr.20707
- JIAN KANG ET AL: "Targeting cyclin-dependent kinase 1 (CDK1) but not CDK4/6 or CDK2 is selectively lethal to MYC-dependent human breast cancer cells", BMC CANCER, BIOMED CENTRAL, LONDON, GB, vol. 14, no. 1, 20 January 2014 (2014-01-20), page 32, XP021174269, ISSN: 1471-2407, DOI: 10.1186/1471-2407-14-32
- S. LIU ET AL: "Inhibition of Rho-Associated Kinase Signaling Prevents Breast Cancer Metastasis to Human Bone", CANCER RESEARCH, vol. 69, no. 22, 3 November 2009 (2009-11-03), pages 8742-8751, XP055665007, US ISSN: 0008-5472, DOI: 10.1158/0008-5472.CAN-09-1541
- C ZHANG ET AL: "ROCK has a crucial role in regulating prostate tumor growth through interaction with c-Myc", ONCOGENE, vol. 33, no. 49, 9 December 2013 (2013-12-09), pages 5582-5591, XP055557620, London ISSN: 0950-9232, DOI: 10.1038/onc.2013.505
- RATTAN et al.: "Rho/ROCK pathway as a target of tumor therapy", Journal of neuroscience research, vol. 83, no. 2, February 2006 (2006-02), pages 243-255, XP055557616,
- ZHANG et al.: "ROCK has a crucial role in regulating prostate tumor growth through interaction with c-Myc", Oncogene, vol. 33, no. 49, December 2014 (2014-12), pages 5582-5591, XP055557620,

## Description

The present invention generally relates generally to a method for identifying a sub-type of cancer sensitive to dimethylfasudil and the use of dimethylfasudil for treating a sub-class of cancerous cells demonstrating high levels of MYC protein expression.

### CROSS-REFERENCE TO RELATED APPLICATIONS

This application claims the benefit of the provisional application entitled MATERIALS AND METHODS FOR EXPLOITING SYNTHETIC LETHALITY IN MYC-OVEREXPRESSING CANCERS with US Application No. 62/514,861 filed on June 4, 2017.

### BACKGROUND OF THE INVENTION

The MYC family's propensity to be mutated and overexpressed contributes to the genesis of many types of human cancers. In fact, the oncogenic MYC family has been found to be deregulated in over 50% of human cancers. The MYC family of oncogenes includes three members, namely C-MYC, MYCN, and MYCL. Collectively, this family of "super-transcription factors" is thought to regulate at least 15% of the entire human genome (and similarly in other animals). Hence many cell processes such as ribosome biogenesis, protein translation, cell-cycle progression, and metabolism are dependent upon proper regulation of the MYC class of genes.

The MYC gene (also known as MYCC or C-MYC) is a proto-oncogene that plays a role in cell cycle progression, apoptosis, and cellular transformation. Many types of cancer cells demonstrate an amplification of the MYC gene. Human colon carcinoma resulting from defects in the Wnt-APC pathway has been found to enhance TCF-mediated transcriptional activation of MYC. In T-cell leukemia, the deregulation of Notch signaling has been associated with inducing the expression of MYC. However, tumorigenesis can also result from the translocation of this gene. For example, Burkitt lymphoma and multiple myeloma are both marked by the translocation of the MYC gene.

Transgenic mouse studies have revealed that even transient disruption of a MYC oncogene is sufficient to elicit tumor regression, suggestion that targeting of the MYC oncogene in cancer therapy may have beneficial effects. Nonetheless, developing a drug that targets the MYC oncogene has proved to be challenging for many reasons. First, MYC is a nuclear protein and so developing specific monoclonal antibodies is technically impractical. Next, unlike kinases, transcription factors lack specific active sites for small molecules to interact with and inhibit transcribing activities.

Kang et al. BMC Cancer 2014, 14:32 shows that targeting cyclin-dependent kinase 1 (CDK1) but not CDK4/6 or CDK2 is selectively lethal to MYC-dependent human breast cancer cells. Liu et al. Cancer Res 2009; 69: (22) shows that inhibition of Rho-associated kinase signaling prevents breast cancer metastasis to human bone. WO 2017/079442 A1 relates to methods of treating a tumor or treating cancer in a subject having a p53 DNA contact mutation that involve administering, to the subject, a ROCK inhibitor. Also disclosed is a method of identifying a subject as a candidate for such treatment. Zhang et al. Oncogene (2014) 33, 5582-5591 shows that ROCK has a crucial role in regulating prostate tumor growth through interaction with c-Myc. Rattan et al. Journal of Neuroscience Research vol. 82, no. 2, 243-255 (2006) relates to Rho/ROCK pathway as a target of tumor therapy.

Accordingly, scientific research has supported many reasons why targeting the MYC oncogene can be an effective strategy in cancer therapy. Nonetheless, it appears that direct targeting of MYC is technically not practicable. Therefore, there is a need in the arts for an effective method for treating cancer cells that overexpress MYC.

### SUMMARY OF THE INVENTION

The present invention is as defined in the claims herein.
The current invention is directed to a method for identifying a sub-type of cancer that responds to inhibition of tumor cell proliferation by means of treatment with dimethylfasudil, comprising the steps of (i) receiving a cancer sample from the subject, (ii) testing for an abnormality of the MYC gene in the sample from the subject, wherein the abnormality of the MYC gene causes higher expression of MYC; and (iii) identifying the type of cancer classification afflicting the subject based on the abnormality found. According to embodiments of this invention, the types of abnormality include, but are not limited to, gene overexpression, detected by comparing the level of MYC gene expression in suspected cancerous cells from the subject to normal cells from the adjoining area, and mutations in a DNA sequence of said MYC gene. In one embodiment, the treatment is administered as a pharmaceutical composition comprising dimethylfasudil.

In another aspect, the invention provides dimethylfasudil for use in treating dimethylfasudil-responsive cancer by: administering an effective dosage amount of dimethylfasudil to cancer cells with an abnormality in MYC gene which cells have been identified as being dimethylfasudil-responsive whereby the dosage amount is sufficient to kill said dimethylfasudil-responsive cancer cells, wherein the abnormality of MYC gene comprises gene overexpression, detected by comparing the level of MYC gene expression in suspected cancerous cells from the subject to normal cells from the adjoining area.

According to embodiments of the claimed inventions, the subject is a mammal, for example a human. In one embodiment, the cancer is Burkitt Lymphoma, Leukemia, or a solid tumor. In one embodiment, the MYC gene is N-MYC. In one embodiment, the abnormality of MYC gene is amplification of MYC by gene duplication. In one embodiment, the abnormality of MYC gene comprises mutations in a DNA sequence of said MYC gene.

According to the preferred embodiment of the current invention, the effective amount of dimethylfasudil is the amount sufficient to inhibit proliferation of cancer cells. In alternate embodiments, the effective amount of dimethylfasudil is the amount which kills the cancerous cells. Yet, in other embodiments, the effective amount of dimethylfasudil is the amount that slows the rate of proliferation.

### BRIEF DESCRIPTION OF THE DRAWING

The patent or application file contains at least one drawing executed in color. Copies of this patent or patent application publication with color drawing(s) will be provided by the Office upon request and payment of the necessary fee.

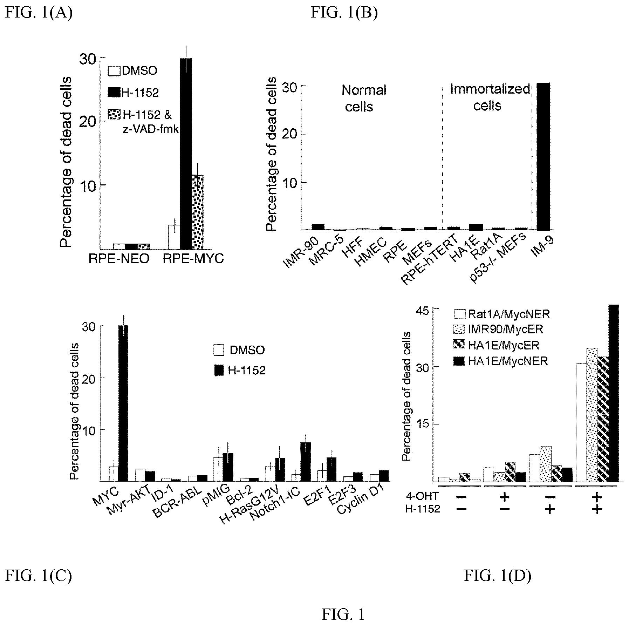

**FIG. 1** demonstrates that H-1152 elicits potent cytotoxicity when MYC is overexpressed. **FIG 1(A)** demonstrates synthetic lethal targeting by H-1152 of human cells overexpressing MYC. Human cells that did overexpress MYC (RPE-MYC) or did not (RPE-NEO) were treated for 72 hours with 0.1% DMSO, 6 µM H-1152, or 6 µM H-1152 + 100 µM z-VAD-fmk. Cell viability was determined by the trypan blue exclusion assay. Each column represents the average of 3 independent experiments, and each experiment was done in triplicate. Error bars represent one standard deviation. **FIG 1(B)** illustrates the cytotoxicity of H-1152 for normal cells and a MYC-overexpressing human cancer cell line IM-9. H-1152 was applied to the indicated cells at 6 µM for 3 days. Cell viability, was determined by the trypan blue exclusion assay. A representative experiment is shown. **FIG 1(C)** shows the toxicity of H-1152 preferentially in rat cells overexpressing Myc. Rat1A cells harboring either an empty vector or various indicated oncogenes were treated with either 0.1% DMSO or 6 µM H-1152 for 3 days. Cell viability was determined by the trypan blue exclusion assay. Each column represents the average of 3 independent experiments, and each experiment was done in triplicate. Error bars represent one standard deviation. **FIG 1(D)** shows synergistic induction of cell death by H-1152 in combination with acute activation of either Myc or MycN. Rat (Rat1A) or human (HA1E and IMR-90) cells expressing either MycER or MycNER were incubated with or without 6 µM H-1152 and/or 200 nM 4-OHT for 3 days. Cell viability was determined by the trypan blue exclusion assay. A representative experiment is shown.

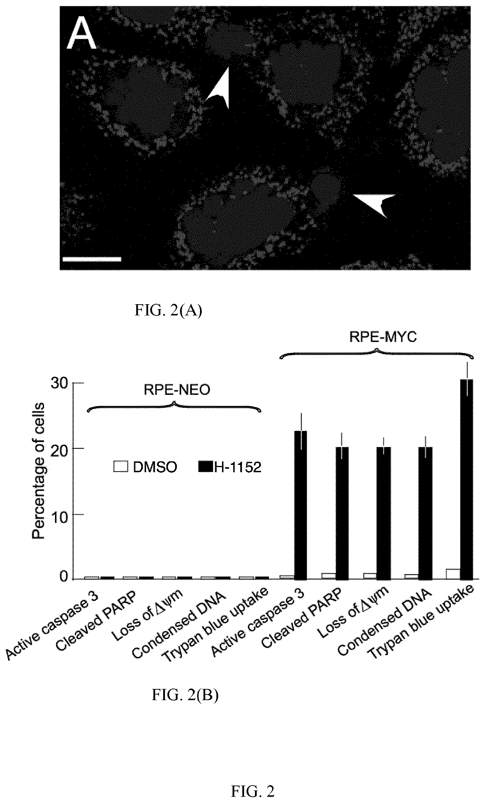

**FIG. 2** demonstrates that H-1152 elicits mitochondria-dependent apoptosis in cells overexpressing MYC. **FIG 2(A)** shows the effect of H-1152 on the mitochondrial membrane potential Δψₘ. RPE-MYC cells were exposed to 6 µM H-1152 for 48 hours and then incubated for 30 minutes with 40 nM MITOTRACKER Red (Molecular Probes) and fixed. Fixed cells were stained for DNA with DAPI (in blue) and then analyzed with a confocal laser microscope. Arrowheads indicate apoptotic nuclei. Scale bar, 10 µm. *See Appendix A* - *for Immunofluorescence Microscopy method.* **FIG 2(B)** shows the percentage of cells positive for indicated assays. RPE-MYC and RPE-NEO cells were treated with 0.1% DMSO or 6 µM H-1152 for 3 days and then subjected to the indicated assays. Each column represents the average of 3 independent experiments, and each experiment was done in triplicate. Error bars represent one standard deviation.

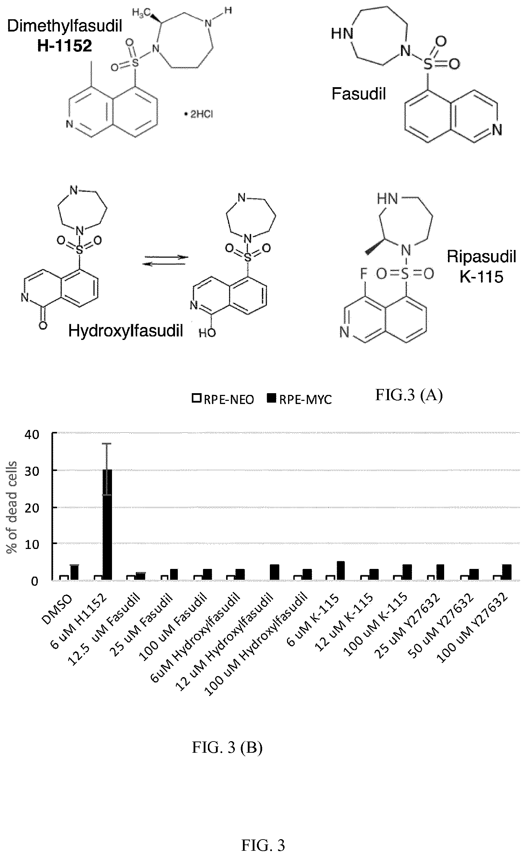

**FIG 3** demonstrates how H-1152 elicits selective killing of MYC-expressing cells independent of its inhibition of ROCK kinase activity. **FIG 3(A)** shows the chemical structure of H-1152 and its three analogs. **FIG 3(B)** illustrates how H-1152, but not other inhibitors of ROCK kinases, preferentially kills RPE-MYC cells. RPE-MYC and RPE-NEO cells were treated for 72 hours with a variety of inhibitors of ROCK kinases at the indicated concentrations. Cell viability was determined by the trypan blue exclusion assay. Each column represents the average of 3 independent experiments, and each experiment was done in triplicate. Error bars represent one standard deviation.

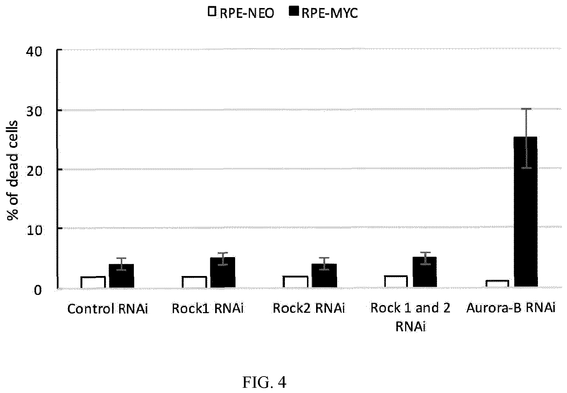

**FIG 4****.** illustrates how the combined depletion of ROCK 1 and ROCK II fails to kill RPE-MYC cells. RPE-NEO and RPE-MYC cells were treated with esiRNA against ROCK1 and ROCK2 either individually or in combination for four days. Aurora-B esiRNA was transfected as a positive control to elicit selectively killing of RPE-MYC as opposed to RPE-NEO cells. Cell viability was then determined by the trypan blue exclusion assay. Cell viability was determined by the trypan blue exclusion assay. Each column represents the average of 3 independent experiments, and each experiment was done in triplicate. Error bars represent one standard deviation.

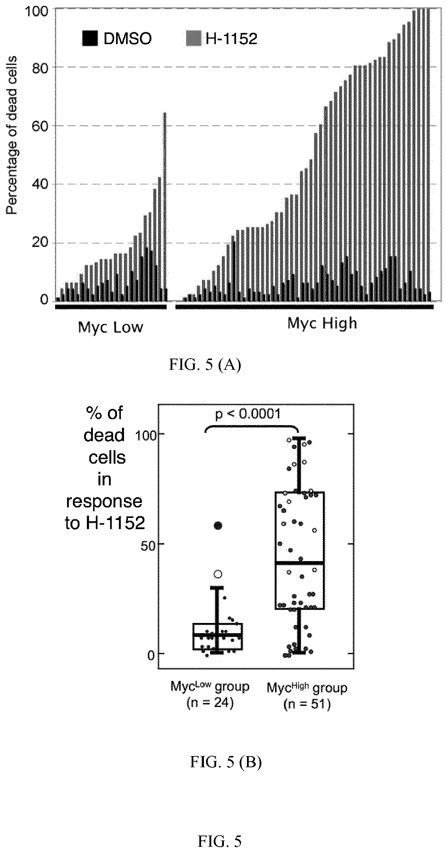

**FIG 5****.** illustrates that MYC abundance is a univariate predicator of the response of human tumor cell lines to H-1152 by showing the cytotoxicity of H-1152 for 75 human cancer cell lines. Cell lines in the Myc^{high} group and the Myc^{low} group were treated for four days with either 0.1% DMSO or 6 µM H-1152. Cell viability was then determined by the trypan blue exclusion assay. **FIG 5(A)** is a histogram representing the average of two independent experiments. **FIG 5(B)** shows a box plot with data swarm displaying the distribution of H-1152 sensitivity in 75 human cancer cell lines. Results are presented as the percentage of dead cells in the presence of H-1152 minus that in the presence of DMSO. Red, yellow and green dots represent cell lines with abundant Myc, MycN and L-Myc respectively. Comparison of the two data sets with two-sided Mann-Whitney test indicates p < 0.0001.

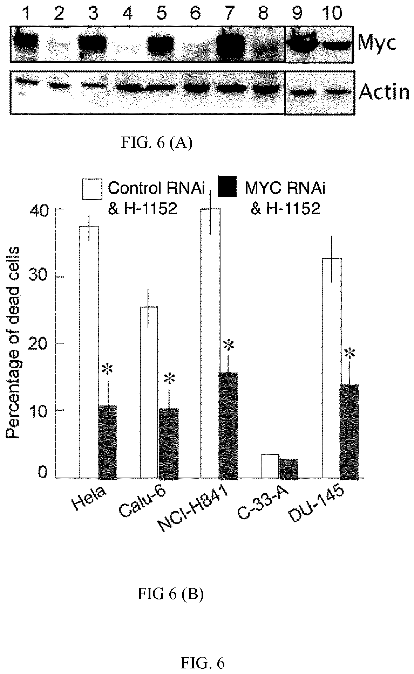

**FIG 6****.** illustrates that MYC abundance in cancer cell lines sensitizes the cells to killing of H-1152. **FIG 6(A)** shows the depletion of MYC in human cancer cell lines by its cognate RNAi. *See Appendix B for RNA Interference Method.* Human cancer cell lines, including Hela (lanes 1 and 2), Calu-6 (lanes 3 and 4), NCI-H841 (lanes 5 and 6), C-33-A (lanes 7 and 8) and DU-145 (lanes 9 and 10), were transfected with either control luciferase esiRNA (lanes 1, 3, 5, 7 and 9) or MYC esiRNA (lanes 2, 4, 6, 8 and 10). Extracts were prepared 3 days after siRNA transfection and immunoblotted for expression of Myc and Actin. **FIG 6(B)** shows the suppression of H-1152 toxicity by depletion of MYC. The indicated cancer cell lines were transfected with either control luciferase esiRNA or MYC esiRNA twice with a 2- day interval and then exposed to 6 µM H-1152 12 hour after the second transfection of siRNA. Cell viability was determined by the trypan blue exclusion assay 4 days after administration of H-1152. Each column represents the average of 3 independent experiments, and each experiment was done in triplicate. Error bars represent standard deviation. Single stars indicate p < 0.001 when compared with cells treated with control RNAi and H-1152. *See Appendix C for more Information on Statistical Analysis.*

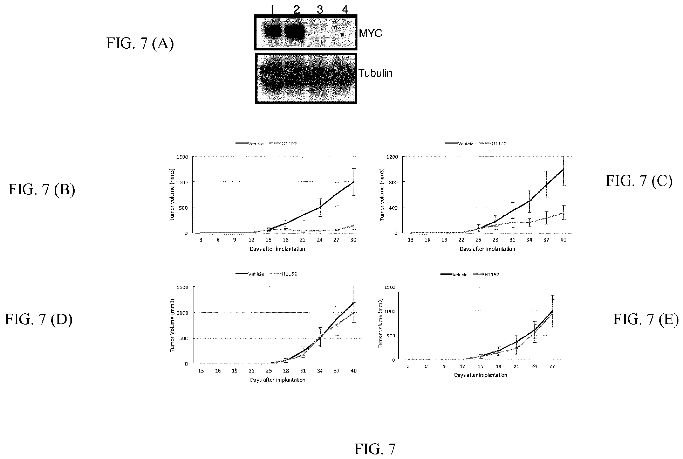

**FIG 7****.** illustrates how H-1152 elicits therapeutic efficacy against tumorigenesis of cancer cells that overexpress MYC. **FIG 7(A)** shows MYC expression in cancer cell lines. Extracts from four human cancer cell lines (1. NCI-H524, 2. NCI-H841, 3. NCI- H211, 4. NCI-H2199) were blotted for MYC and tubulin by Western blotting. FIG 7(B) - (E) demonstrate the therapeutic efficacy of H-1152 *in vivo. See Appendix D for Method of Mouse In Vivo Assay.* Two Myc^{high} cell lines and two Myc^{low} cell lines were implanted subcutaneously into athymic mice (One to ten millions of cells per injection). When tumors reached approximately 50-70 mm³, tumor-bearing mice were randomized to receive oral gavage with either vehicle of H-1152 (60 mg/kg body weight per injection) twice a day until the endpoint. The tumor volume was determined at the indicated time points. **FIG 7(B)** shows results for NCI-H524; **FIG 7(C)** shows results for NCI-H841; **FIG 7(D)** shows results for NCI-H2199; and **FIG 7(E)** shows results for NCI-H211.

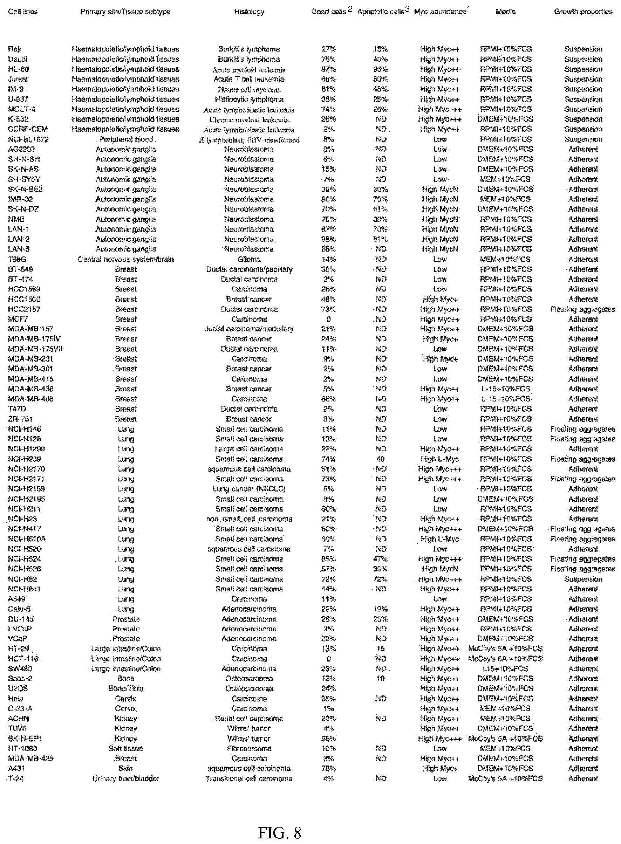

**FIG 8****.** is a table summarizing MYC abundance and responses of human cancer cell lines.

### DETAILED DESCRIPTION OF THE INVENTION

The invention relates to the use of an inhibitor of Rho-associated protein kinase (hereinafter "**ROCK**") in methods for treatment of cancer cells. Specifically, the method is directed at treatment of cancer cells, especially those cells that exhibit high expression levels of the MYC oncogene using dimethylfasudil. The contemplated method herein can be used alone or in tandem with traditional cancer treatments such as chemotherapy and/or radiation therapy.

ROCK is a family of kinases comprising of the homologues ROCK 1 and ROCK 2 that belong to the AGC (PKA/PKG/PKC) family of serine-threonine kinases. As a downstream effector protein of the GTPase Rho, ROCK is major regulator of the cytoskeleton. Based on its roles in actin depolymerization, contraction of actin fibers, and filament stabilization, ROCK activity is crucial for proper cell migration.

ROCK 1 and ROCK 2 demonstrate significant homology between themselves, sharing over 65% identity in their amino acid sequences. Significantly, ROCK1 and ROCK2 have the highest amino acid homology between their kinase domain (92%), resulting in similar kinase activity in vitro. However, the PH-C1 domains of ROCK1 and ROCK2 have differential binding preferences for membrane lipids: the PH-C1 domain of ROCK2 was demonstrated to bind strongly to phosphatidylinositol (3,4,5)-trisphosphate and phosphatidylinositol (4,5)-bisphosphate, whereas the one of ROCK1 did not. This difference in binding potential may suggest why ROCK 1 and ROCK 2 have distinct subcellular distributions, which vary depending on the cell type and method, and why they have overlapping but diverging functions as well.

The invention is based on the finding that the ROCK 1 and ROCK 2 inhibitor dimethylfasudil (hereinafter "**H-1152**") elicited cytotoxicity in various cells overexpressing MYC. H-1152 is a potent, selective, and reversible inhibitor of ROCK 1 and ROCK 2 that was developed to treat neurological disorders and cardiovascular diseases. Nonetheless, a screening of various small molecules that induce lethal interactions in cells overexpressing MYC has revealed that H-1152 is effective in eliciting cell death in MYC overexpressing cell lines such as IM-9 but fails to have similar lethal effects in normal and immortalized cells.

An aspect of the present invention is directed to H-1152 for use in a method of treating H-1152-responsive cancer (and consequently inducing cell toxicity, inhibiting cell proliferation and/or survival) in an effective amount. In one embodiment, this method involves administering H-1152 to a population of cancer cells.

In one embodiment of the present invention, the population of cancer cells targeted for treatment by H-1152 are haematopoietic lymphoid tissues. Hematological malignancies account for 9.5% of new cancer diagnoses in the United States. Tumors of hematopoietic and lymphoid tissues affect members of the circulatory and immune system, namely the blood, bone morrow, lymph, and lymphatic system. A common cause of cancer in such systems is chromosomal translocations. One embodiment of the present invention is directed to Burkitt's lymphoma. This type of cancer targets the lymphatic system generally but especially B lymphocytes. Though there are various types of Burkitt lymphoma, all are characterized by the dysregulation of the MYC gene due to chromosomal translocations. The typical translocation of MYC into the immunoglobulin heavy chain locus is observed in about 80% of Burkitt's lymphomas. Other variant translocations of MYC involve either the κ or λ light chain locus, with type each type of translocation occurring at a frequency of about 10% of all Burkitt's lymphoma cases. Another embodiment of the present invention is directed to leukemia, including but not limited to acute T-cell leukemia, where the MYC gene is observed to be translocated into one of the T cell receptor loci. Other embodiments of the current invention are directed to acute myeloid leukemia, acute lymphoblastic leukemia, chronic myeloid leukemia, acute lymphoblastic leukemia, neuroblastoma, and ductal carcinoma.

In another embodiment of the present invention, the population of cancer cells are derived from a solid tumor, such as sarcomas (e.g. osteosarcoma), carcinomas (e.g. adenocarcinoma from the prostrate; small cell carcinoma of the lung carcinoma of the breast), Wilms' tumor, squamous cell carcinoma, and lymphomas.

According to some embodiments of the current invention, a determination is made as to whether the tumor cells overexpress the MYC oncogene. Overexpression can be defined in multiple ways, but at its most essence, it is defined as the level of gene expression above normal. It has been known that the gene expression patterns are complexly different between normal and cancerous cells. Accordingly normal expression of gene is defined as the level of protein that does not induce a noticeable change whereas overexpression of a gene is defined as such level that contributes to phenotypic variability.

There are primarily two means by which a gene can be overexpressed. In one simplified model, a gene is overexpressed due to allosteric reactions. The other is due to gene duplication.

The measurement of gene overexpression is accomplished by many different methods and techniques. One method is to compare the mRNA levels of genes in normal human tissue (preferably of identical age, sex, and ethnicity to minimize differing factors) with those of the patient. The mRNA levels can be quantified and compared using northern blotting or RT-qPCR. Another method is to measure the level of protein quantification using techniques such as Western blotting. However, the foregoing methods are only presented for the purpose of illustration. One of ordinary skill in the art can utilize any or more of the multiple qualitative and quantitative techniques to determine whether the MYC gene is being overexpressed in a patient. The following is one example (with select alternatives) for determining MYC expression by cell isolation in the patient.

### ISOLATING TISSUE CELLS FOR MYC ANALYSIS

First a biopsy is performed to extract cancerous primary tissue samples from the patient. The tissue sample is treated with proteolytic enzymes (e.g. trypsin and collagenase) to disrupt the extracellular matrix holding the tissue sample together. Preferably, the tissue samples are further treated with agents such as ethylenediaminetetraacetic acid ("EDTA") to chelate calcium ions in order to disrupt the cell-cell adhesion. The tissue is then dissociated into single living cells by gentle agitation. The cell suspension is further refined by exploiting the specific binding properties of antibodies. Antibodies that bind specifically to surface proteins are coupled to matrices such as collage, polysaccharide beads, plastic, and fluorescent dye that are further exploited to isolate the cells. Using fluorescent dye as a label, the cells are sorted from unlabeled cells via an electronic fluorescence-activated cell sorter.

Another method for isolating cells is to perform laser microdissection. This method entails the careful dissection of thin tissues slices prepared for microscopy. In one approach the cancerous tissue section is coated with a thin plastic film. The area containing the selected cells is irradiated with a focused infrared laser beam pulse. Consequently, the area is melted, causing cell binding underneath. The captured cells are then removed for additional analysis.

### QUANTITATIVE DETERMINATION OF MYC ABUNDANCE

Once the cancerous tissue cells are isolated, a quantitative analysis of the abundance of MYC protein is performed to determine whether the cancer cells are suitable for the claimed treatment. One method to perform such analysis is to compare MYC expression in the tissue cells with cell lines stably introduced with MYC selection marker in combination with hTERT in human retinal pigment epithelial cells. *See Appendix E for Preparation of MYC stabilized cells.*

Another method to measure MYC expression is to isolate the tumor cells from the adjoining normal cells and measure. There are significant phenotypic differences in the appearance of normal cells and cancer cells. Unlike normal cells, cancer cells have abnormal shape due to their inability to stop growing. Accordingly, the cancerous cells keep doubling, forming a tumor. Since cancer cells are apparent against a backdrop of normal cells, each type of cells - tumor cells and normal cells, can be isolated for quantifying gene expression and. As an example, if a patient has a liver cancer, the level of MYC expression in the tumor liver cells is compared with the level of MYC expression of the adjoining but normal cells. Similarly, if a patient suffers from breast cancer in one breast, the level of MYC expression from the breast tumor on breast 1 can be measured against normal MYC expression from the unaffected breast 2.

Upon harvesting suspected cancerous cells along with a control group (e.g. normal cells from cell line or normal cells from adjoining area) for analysis, expression of MYC can be determined by Western blotting analysis. *See Appendix F for Western Blot Protocol.* Cancer cells that express comparable levels of MYC are selected as candidates for the claimed invention.

### TREATMENT WITH H-1152

Once candidates are selected, they may be treated with H-1152. The H-1152 may be freshly dissolved into vehicle (10% DMSO, 90% polyethylene glycol 300) and administered by oral gavage twice every day.

In therapeutic and/or diagnostic applications, the compound for use in the invention can be formulated for a variety of modes of administration, including systemic and topical or localized administration. Techniques and formulations generally may be found in Remington: The Science and Practice of Pharmacy (20th ed.) Lippincott, Williams & Wilkins (2000).

According to embodiments of the invention, H-1152 is effective over a wide dosage range. For example, in the treatment of adult humans, dosage can range from 0.01 mg to 1000 mg. Although contingent on multiple factors, a preferable dose is 10 mg to 30 mg of H-1152 per day. The exact dosage will depend upon the route of administration, the form in which the compound is administered, the subject to be treated, the body weight of the subject to be treated, and the preference and experience of the attending physician.

In another embodiment of the current invention, a method of treating cancer cells involves targeting the MYC gene with H-1152 in an amount. Certain cancer types are marked by identifiable mutations. For example, mutations in Thr-58 and Ser-62 of MYC are present in a prevalent type of Burkitt lymphoma. These mutations are associated with stabilized mutant proteins that can perturb transgenic mammary tumorigenesis. In such case when the mutations in the MYC gene are identified, treatment can be effected by H-1152.

Similarly an alternate embodiment of the current invention claims a method for identifying a cancer sub-type that responds to H-1152 by receiving a cancer sample from a subject, testing for an abnormality of the MYC gene, wherein the abnormality of the MYC gene causes higher expression of MYC, and then using the results of the test to decipher the type of cancer sub-type afflicting the subject. For example, a cancer patient may be treated with H-1152 and it is noticed that the tumor has either stopped progressing or has regressed. According to this alternate embodiment of the claimed invention, the tumor cells can be harvested and assayed for MYC analysis since they show a response to H-1152. Once assayed, it can be determined whether the MYC gene is altered, whether by mutation or translocation (or both). Such information can be used for diagnostic purposes or to refine treatment.

The following section provides experimental support for the use of H-1152 in cancer treatment.

### RESULTS - SELECTIVE CYTOTOXICITY IN MYC-OVEREXRESSING CELLS

In order to confirm that H-1152 elicits cytotoxicity in cells overexpressing MYC, human retinal pigment epithelial cells were stably transfected with either MYC or a Neomycin selection marker gene **("NEO")** in combination with hTERT. After a 3-day treatment with H-1152, 30% of cells stably transfected with the MYC gene were killed whereas none of the cells transfected with the NEO gene were killed. A separate control experiment testing for the cytotoxicity of the H-1152 solvent demonstrated that the solvent failed to elicit any cell death in either the MYC-expressing or NEO-expressing cells. **FIG 1A****.**

Since the aforementioned effects of H-1152 were observed in artificially induced MYC expressing gene, the effects of H-1152 were evaluated in a variety of normal cells and immortalized cells. The result after 3-days treatment were similar. In detail, of the cell lines evaluated, H-1152 has negligible effect on cell toxicity regardless of whether the cells lines are normal cells or immortalized cells. The one observed exception is the IM-9 cells that overexpress MYC. Cell lethality in the IM-9 cell line was approximately 30% after H-1152 treatment for 3 days. **FIG 1B****.**

To determine whether cytotoxicity by H-1152 is dependent on MYC-expression or any oncogene, rat 1A cells expressing MYC as well as other active cellular oncogenes were evaluated for lethality. Among the different cells with various active oncogenes, only MYC expressing cells demonstrated cell death upon treatment by H-1152. **FIG 1C****.**

### H-1152 IS EVEN EFFECTIVE UPON ACUTE ACTIVATION OF MYC

In order to negate the possibility that sustained expression of MYC gene has given rise to unidentified targets affected by H-1152, sensitivity of H-1152 in acutely activated cells was measured. Acute activation of MYC was exploited by means of a MYC-estrogen receptor chimera. This receptor is rapidly activated by the addition of 4-hydroxy-tamoxifen ("4-OH"). While both 4-OH and H-1152 in themselves resulted in low levels of cell death, collectively the drugs acted synergistically to elicit over 30% cell lethality in both human lung fibroblasts (IMR-90) and renal epithelial HA1E cell lines (HA1E). **FIG 1D****.** A similar experiment was conducted with acute activation of the MYCN gene. MYCN shares high sequence similarity and crucial biochemical and genetic activities with MYC. As with MYC expression, activation of the MYCNestrogen receptor chimera with 4-OHT resulted in high cell lethality upon H-1152 application. Accordingly, H-1152 elicits MYC-dependent cell lethality irrespective of whether MYC is constitutively expressed or acutely activated. Furthermore, this sensitivity holds true for both fibroblasts cells and epithelial cells.

### H-1152 INDUCED CELL LETHALITY ELICITS APOPTOSIS

To determine the mechanism by which cell death is induced in MYC expressing cells treated with H-1152, the treated cells were observed for apoptotic features. Symptoms of classical apoptosis were recorded, such as nuclear condensation, activation of caspase 3, and cleavage caspase 3's downstream target, poly (ADP-ribose) polymerase ("PARP"). **FIG 2B****.** Furthermore, observations of events such as the dissipation of the mitochondrial membrane potential, release of cytochrome C from mitochondria into the cytosol, and the activation of caspase 9 all indicate cell death via activation of the mitochondria-dependent apoptosis pathway. **FIB 2A.** To further support the observations that H-1152 promotes mitochondria-dependent apoptosis pathway, overexpressing MYC cells were treated with both H-1152 and pancaspase inhibitor z-VAD-fmk. While H-1152 alone elicited approximately 30% lethality in treated cells, the addition of the pancaspase inhibitor reduced cell lethality by approximately half, resulting in 15% cell lethality. Since apoptosis was effectively inhibited, the level of cell death was also reduced. Accordingly, H-1152 promotes cell death by activation of the mitochondria-dependent apoptosis pathway.

### H-1152 INDUCED LETHALITY IS INDEPENDENT OF ROCK INHIBITION

To confirm that the lethality imparted by H-1152 in overexpressing MYC cancer cells is due to inhibition of ROCK kinase as opposed to an unintended target, the effects of H-1152 were compared with five other ROCK inhibitors. Among of the five, fasudil, hydroxylfasudil and rapasudil (K-155) are analogs of H-1152. Fasudil has an IC₅₀ of 330 nM for ROCKII, hydroxylfasudil has a IC₅₀ of 720 nM and 730 nM for ROCKI and ROCKII, and Rapasudil (K-155) has a IC₅₀ of 51 nM and 19 nM for ROCKI and ROCK II. (Figure 3A). For the remaining two, Y-27634 has an IC₅₀ of 140 nM and 300 nM for ROCKI and ROCKII respectively. GSK429286A/RHO-15 has an IC₅₀ for ROCKI and ROCK II 14 nM and 63 nM respectively.

A measurement of lethality in cells transfected with RPE-MYC demonstrates that despite all five agents being ROCK inhibitors, only treatment with H-1152 showed significant cell death. **FIG. 3B****.** In fact even a 15-fold increase of these inhibitors to 100µM failed to kill the RPE-MYC transfected cells. **FIG 3B****.** Such data leads to the finding that MYC-dependent lethality elicited by H-1152 is not mediated by the inhibition of Rho kinases.

To support this finding, expression of ROCK I and ROCK II were inhibited by RNAi in the RPE-MYC cells. If H-1152 did elicit cell toxicity by inhibiting ROCK 1 and/or ROCK II, then a similar cell toxicity profile would result with RNAi. However, inhibition of ROCK 1 and ROCK II with RNAi failed to yield similar lethality; rather over 90% of the RPE-MYC cells survived despite inhibiting the expression of ROCK 1 and/or ROCK II. **FIG. 4****.** Accordingly, MYC-dependent cytotoxicity of H-1152 is not attributed to the disabling and inhibition of ROCK 1 and ROCK 11.

### MYC ABUNDENCE POSITIVELY CORRELATES WITH H-1152 SENSITVITY

In order to determine whether human cancer cell sensitivity to H-1152 correlates with MYC expression, forty (40) cell lines exhibiting proximate to or higher expression of MYC relative to RPE-MYC cells ("**Myc^{high}**") were compared with 24 cell lines exhibiting proximate to or less than RPE-NEO cells ("**Myc^{low}**"). *See* **FIG 8****.** The Myc^{high} group also includes 8 cell lines with abundant MycN expression and 3 cell lines abundantly expressing L-Myc. Treatment with 6 µM H-1152 for 4 days elicited massive cell death in a much higher percentage of cell lines in the Myc^{high} group than that in the Myc^{low} group. **(****Figure 5** **A, B).** The difference between the two data sets was statistically significant.

While there is a positive correlation between H-1152 sensitivity and MYC expression, there remain some outliers. Results indicate that 3 of 24 cell lines in the Myc^{low} group were nonetheless sensitive to H-1152 suggesting that oncogenic elements other than the overexpressed MYC may be conferring cells to H-1152 sensitivity **(****Fig. 5** **A, B).** In contrast, 11 of the 51 cell lines in the Myc^{high} group failed to undergo apoptosis when treated with H-1152, indicating that additional lesions might have suppressed MYC-dependent apoptosis elicited by H-1152 **(****Fig. 5** **A, B)**.

### DEPLETION OF MYC MODULATES CYTOTOXIC SENSITIVITY TO H-1152

To confirm whether abundant MYC in human cancer cells is responsible for H-1152 sensitivity, expression of MYC was blocked with cognate siRNA in select Myc^{high} cancer cell lines, namely Hela, NCI-H841, C-33-A, Calu-6 and MDA-MB-157. Depletion of MYC alone failed to elicit any appreciable levels of toxicity upon H-1152 treatment. Rather depletion of MYC provided 50% to 75% protection at 96 hours against the cytotoxicity elicited by H-1152. **(****Fig. 6B****).** Accordingly, these results demonstrate that abundant MYC express is required in human cancer cell lines to induce cell apoptosis in response to H-1152.

### H-1152 EFFECTIVE AGAINST TUMORS WITH ABUNDANT MYC EXPRESSION

To determine whether tissue culture results can be replicated in vivo, two cell lines from Myc^{High} group, namely NCI-H841 and NCI-H524, and two cell lines from Myc^{Low} group, namely NCI-H211 and NCI-H2199. All four cell lines were able to form xenograft tumors in athymic mice upon implanted subcutaneously. Upon detection of visible tumor, the mice were randomized to receive either H-1152 or vehicle. Mice implanted with MYC^{High} cells either stabilized tumor development **(****FIG 7B****)** or slowed down tumor development **(****FIG 7C****).** In contrast, H-1152 failed to impact tumor development in cell lines from the Myc^{Low} group **(****FIG 7D**, E). These results collectively demonstrate that tumor cells demonstrating high levels of MYC expression can be treated with H-1152. Furthermore, overexpression of a MYC family oncogene or a molecular signature of MYC target genes can serve as predicative biomarkers for sensitivity to H-1152 and its analogs.

### POTENTIAL ANALOGS

In assessing the efficacy of H-1152 in cell lethality, it was determined that both methyl groups are essential for H-1152 to elicit MYC-dependent cytotoxicity. Accordingly, other embodiments of the present invention include treatment of MYC-expressing tumor cells with H-1152 with modifications in one or both of the methyl groups.

While multiple embodiments are disclosed, still other embodiments of the present invention will become apparent to those skilled in the art from this detailed description. Accordingly, the drawings and descriptions are to be regarded as illustrative in nature and not restrictive.

### APPENDIX A - IMMUNOFLUROESCENCE MICROSCOPY

Cells are cultured on coverslips in a 6-well plate, fixed with 4% paraformaldehyde or 4% paraformaldehyde followed with methanol treatment, and then permeabilized with 0.3% Triton X-100. Rabbit polyclonal antibodies for active caspase 3, active caspase 9 and cleaved PARP from Cell Signaling Technology can be used. Primary antibodies are detected with Texas red-conjugated or fluorescein isothiocyanate- conjugated secondary antibodies that may be purchased from Jackson ImmunoResearch. After immunostaining, cells are mounted on microscope slides with 4',6'-diamidino-2- phenylindole (DAPI)-containing Vectashield mounting solution (Vector Laboratories).

For determination of mitochondrial membrane potential, cells are cultured on cover slips and exposed for 30 min at 37^{O}C with 40 nM Mitotracker# Red CMXRos (Molecular Probes), a dye that accumulates in mitochondria as a function of the mitochondrial potential. After fixation in 4% paraformaldehyde, the cells are mounted on microscope slides with DAPI-containing Vectashield mounting solution from Vector Laboratories. For fluorescence detection, an EVOS FL auto microscope (THERMO FISHER) can be used.

### APPENDIX B - RNA INTERFERENCE

MYC, Firefly Luciferase, ROCK1 and ROCK2 esiRNA is generated by digestion of the double-stranded RNA corresponding to the full-length coding sequence of the cognate genes with *E. coli* RNase III. EsiRNAs smaller than 30 bp is purified with DEAE columns and is transfected into cells with reagents such as LIPOFECTAMINE 2000 (INVITROGEN) according to the manufacturer's instructions.

### APPENDIX C - STATISTICAL ANALYSIS

Statistical analyses is performed with the GRAPHPAD PRISM software. Statistical significance of the differences was evaluated with Student's unpaired two- tailed t test. P values less than 0.05 were considered statistically significant.

### APPENDIX D - MOUSE IN VIVO PROTOCOL

Experimental mice are housed and treated according to the protocol approved by the Institutional Animal Care and Use Committee of MBICR. To create tumor- bearing mice for the treatment experiments, one to ten million human cancer cells in phosphate buffered saline (PBS) are injected subcutaneously into the right flank of each athymic mice. When tumor volumes of the majority of mice reach around 50 mm³, tumor-bearing mice are randomized to receive either vehicle or H-1152 in a double- blinded manner. A cohort of ten mice are used for each group. H-1152 was freshly dissolved into vehicle (10% DMSO, 90% polyethylene glycol 300) and administered by oral gavage twice every day (60 mg/kg body weight per treatment). Tumor diameter at injection sites are monitored at the indicated time points with a digital caliper and the volume calculated using the formula: V (mm3) = A x B x B/2, where A and B represent the largest and smallest diameters of the tumor respectively.

### APPENDIX E - PREPARATION OF MYC STABLIZED CELL LINES

Human primary cells and tumor cell lines can be obtained from the AMERICAN TYPE CULTURE COLLECTION. RPE-NEO and RPE-MYC cells are generated by transfecting human primary retinal pigment epithelial cells with hTERT in combination with either Neomycin resistant gene or the MYC oncogene and then selected with 1 ug/ml Puromycin.

### APPENDIX F - WESTERN BLOT PROTOCOL

Whole cell extracts are prepared by incubating cells for 15 min at 4 °C in a lysis buffer [50 mM Tris (pH 7.5), 200 mM NaCL, 0.1% SDS, 1% Triton X-100, 0.1 mM DTT, and 0.5 mM EGTA] supplemented with protease inhibitor mixture. The extracts are centrifuged at 8,000 × g for 10 min to clear insoluble material. The protein concentration in the supernatant is determined using a protein assay. Lysate containing 50-100 µg of proteins are resolved on NuPAGE (4-12%) Bis-Tris gels and transferred to nitrocellulose membranes. The membranes are blocked with 5% nonfat milk in PBS buffer for 1 h and then incubated overnight at 4 °C with primary antibodies diluted 1:1,000 in the blocking buffer. Rabbit polyclonal antibodies are used for MYC, MYCN and Tubulin.

## Claims

1. A method for identifying a sub-type of cancer responding by inhibition of tumor cell survival to dimethylfasudil treatment in a subject in need thereof, comprising the steps of:
(a) receiving a cancer sample from the subject;
(b) testing for an abnormality of MYC gene in the sample from the subject; wherein the abnormality of the MYC gene causes higher expression of MYC; and
(c) identifying in the subject a sub-type of cancer sensitive to dimethylfasudil treatment according to the detection of the MYC abnormality in the sample provided by the subject.

2. The method of claim 1, wherein the abnormality of MYC gene comprises gene overexpression, detected by comparing the level of MYC gene expression in suspected cancerous cells from the subject to normal cells from the adjoining area.

3. The method of claim 1, wherein the abnormality of MYC gene comprises of mutations in a DNA sequence of said MYC gene.

4. The method of claim 1, wherein the treatment is administered as a pharmaceutical composition comprising dimethylfasudil.

5. Dimethylfasudil for use in treating dimethylfasudil-responsive cancer by:
administering an effective dosage amount of dimethylfasudil to cancer cells with an abnormality in MYC gene which cells have been identified as being dimethylfasudil-responsive whereby the dosage amount is sufficient to kill said dimethylfasudil-responsive cancer cells, wherein the abnormality of MYC gene comprises gene overexpression, detected by comparing the level of MYC gene expression in suspected cancerous cells from the subject to normal cells from the adjoining area.

6. Dimethylfasudil for use according to claim 5, wherein the subject is a mammal.

7. Dimethylfasudil for use according to claim 5, wherein the subject is human.

8. Dimethylfasudil for use according to claim 7, wherein the cancer is Burkitt Lymphoma.

9. Dimethylfasudil for use according to claim 7, wherein the cancer is Leukemia.

10. Dimethylfasudil for use according to claim 7, wherein the cancer is a solid tumor.

11. Dimethylfasudil for use according to claim 5, wherein the MYC gene is N-MYC.

12. Dimethylfasudil for use according to claim 5, wherein the abnormality of MYC gene is amplification of MYC by gene duplication.

13. The method of claim 1, wherein the abnormality of MYC gene comprises mutations in a DNA sequence of said MYC gene.

## Patentansprüche

1. Verfahren zur Identifizierung einer Krebsunterart, die durch Hemmung von Tumorzellüberleben auf eine Dimethylfasudilbehandlung bei einem diese benötigenden Individuum anspricht, umfassend die Schritte:
(a) Empfangen einer Krebsprobe von dem Individuum;
(b) Testen auf eine MYC-Gen-Anomalie in der Probe vom Individuum; wobei die Anomalie des MYC-Gens eine höhere Expression von MYC gestattet; und
(c) Identifizieren einer Krebsunterart beim Individuum, die gegenüber Dimethylfasudilbehandlung empfindlich ist, gemäß dem Nachweis der der MYC-Anomalie in der vom Individuum bereitgestellten Probe.

2. Verfahren nach Anspruch 1, wobei die MYC-Gen-Anomalie Genüberexpression, nachgewiesen durch Vergleichen des MYC-Genexpressionsniveaus in verdächtigen kanzerösen Zellen von dem Individuum mit Normalzellen aus dem angrenzenden Bereich, umfasst.

3. Verfahren nach Anspruch 1, wobei die MYC-Gen-Anomalie Mutationen in einer DNA-Sequenz des MYC-Gens umfasst.

4. Verfahren nach Anspruch 1, wobei die Behandlung in Form einer Dimethylfasudil umfassenden pharmazeutischen Zusammensetzung verabreicht wird.

5. Dimethylfasudil zur Verwendung beim Behandeln einer auf Dimethylfasudil ansprechenden Krebserkrankung durch: Verabreichen einer wirksamen Dosierungsmenge Dimethylfasudil an Krebszellen mit einer Anomalie im MYC-Gen, wobei die Zellen als auf Dimethylfasudil ansprechend identifiziert wurden, wobei die Dosierungsmenge zum Abtöten der auf Dimethylfasudil ansprechenden Krebszellen hinreichend ist, wobei die MYC-Gen-Anomalie Genüberexpression, nachgewiesen durch Vergleichen des MYC-Genexpressionsniveaus in verdächtigen kanzerösen Zellen von dem Individuum mit Normalzellen aus dem angrenzenden Bereich, umfasst.

6. Dimethylfasudil zur Verwendung gemäß Anspruch 5, wobei es sich bei dem Individuum um einen Säuger handelt.

7. Dimethylfasudil zur Verwendung gemäß Anspruch 5, wobei es sich bei dem Individuum um einen Menschen handelt.

8. Dimethylfasudil zur Verwendung gemäß Anspruch 7, wobei es sich bei der Krebserkrankung um Burkitt-Lymphom handelt.

9. Dimethylfasudil zur Verwendung gemäß Anspruch 7, wobei es sich bei der Krebserkrankung um Leukämie handelt.

10. Dimethylfasudil zur Verwendung gemäß Anspruch 7, wobei es sich bei der Krebserkrankung um einen soliden Tumor handelt.

11. Dimethylfasudil zur Verwendung gemäß Anspruch 5, wobei es sich bei dem MYC-Gen um N-MYC handelt.

12. Dimethylfasudil zur Verwendung gemäß Anspruch 5, wobei es sich bei der MYC-Gen-Anomalie um Amplifikation von MYC durch Genduplikation handelt.

13. Verfahren nach Anspruch 1, wobei die MYC-Gen-Anomalie Mutationen in einer DNA-Sequenz des MYC-Gens umfasst.

## Revendications

1. Procédé pour l'identification d'un sous-type de cancer répondant par inhibition de la survie de cellules tumorales à un traitement par le diméthylfasudil chez un sujet qui en a besoin, comprenant les étapes de :
(a) réception d'un échantillon cancéreux provenant du sujet ;
(b) test d'une anomalie du gène MYC dans l'échantillon provenant du sujet ; l'anomalie du gène MYC causant une expression plus élevée de MYC ; et
(c) identification chez le sujet d'un sous-type de cancer sensible à un traitement par le diméthylfasudil selon la détection de l'anomalie MYC dans l'échantillon mis à disposition par le sujet.

2. Procédé selon la revendication 1, l'anomalie du gène MYC comprenant une surexpression génique, détectée en comparant le niveau d'expression du gène MYC dans des cellules suspectées d'être cancéreuses provenant du sujet à des cellules normales provenant de la zone adjacente.

3. Procédé selon la revendication 1, l'anomalie du gène MYC comprenant des mutations dans une séquence d'ADN ou dudit gène MYC.

4. Procédé selon la revendication 1, le traitement étant administré comme une composition pharmaceutique comprenant du diméthylfasudil.

5. Diméthylfasudil pour une utilisation dans le traitement d'un cancer sensible au diméthylfasudil par :
administration d'une quantité de dosage efficace de diméthylfasudil à des cellules cancéreuses comportant une anomalie du gène MYC, lesquelles cellules ont été identifiées comme étant sensibles au diméthylfasudil, moyennant quoi la quantité de dosage est suffisante pour détruire lesdites cellules cancéreuses sensibles au diméthylfasudil, l'anomalie du gène MYC comprenant une surexpression génique, détectée en comparant le niveau d'expression du gène MYC dans des cellules suspectées d'être cancéreuse provenant du sujet à des cellules normales provenant de la zone adjacente.

6. Diméthylfasudil pour une utilisation selon la revendication 5, le sujet étant un mammifère.

7. Diméthylfasudil pour une utilisation selon la revendication 5, le sujet étant un être humain.

8. Diméthylfasudil pour une utilisation selon la revendication 7, le cancer étant un lymphome de Burkitt.

9. Diméthylfasudil pour une utilisation selon la revendication 7, le cancer étant une leucémie.

10. Diméthylfasudil pour une utilisation selon la revendication 7, le cancer étant une tumeur solide.

11. Diméthylfasudil pour une utilisation selon la revendication 5, le gène MYC étant N-MYC.

12. Diméthylfasudil pour une utilisation selon la revendication 5, l'anomalie du gène MYC étant une amplification de MYC par duplication génique.

13. Procédé selon la revendication 1, l'anomalie du gène MYC comprenant des mutations dans une séquence d'ADN dudit gène MYC.
